# EUROPEAN PATENT SPECIFICATION

(11) **EP 0 662 961 B1**
(45) Date of publication and mention of the grant of the patent: **10.11.1999**
(21) Application number: 93921386.4
(22) Date of filing: 07.09.1993
(51) Int. Cl.: C07D 233/64, C07D 233/90, C07D 233/54

(54) **PROCESS OF SYNTHESIZING USEFUL INTERMEDIATES OF SUBSTITUTED IMIDAZOLE COMPOUNDS**
VERFAHREN ZUR SYNTHESE NÜTZLICHER ZWISCHENPRODUKTE VON SUBSTITUIERTEN IMIDAZOLVERBINDUNGEN
PROCEDE DESTINE A SYNTHETISER DES INTERMEDIAIRES UTILES DE COMPOSES D'IMIDAZOLE SUBSTITUES

(30) Priority: 23.09.1992 GB 9220068
(43) Date of publication of application: 19.07.1995
(73) Proprietor: SMITHKLINE BEECHAM CORPORATION, Philadelphia Pennsylvania 19101 (US)
(72) Inventor: MOKHALLALATI, Mohamed Kheir, King of Prussia, PA 19406 (US); PRIDGEN, Lendon, Norwood, Kind of Prussia, PA 19406 (US); SHILCRAT, Susan, King of Prussia, PA 19406 (US); WEINSTOCK, Joseph, King of Prussia, PA 19406 (US)
(74) Representative: Connell, Anthony Christopher
(86) International application number: US9308390
(87) International publication number: WO9406776

(56) References cited:
- US-A- 4 340 598
- US-A- 4 482 723
- US-A- 4 602 093
- US-A- 5 075 452
- US-A- 5 234 917

## Description

The present invention relates to a process for preparing useful intermediates in the synthesis of substituted imidazole compounds. Such compounds are described in EP-A-0403159 as being angiotensin II receptor antagonists useful in the treatment of hypertension, congestive heart failure, renal failure, and glaucoma.

### BACKGROUND OF THE INVENTION

EP-A-0403159 describes a process for the preparation of imidazole intermediates which comprises a high pressure liquid ammonia condensation of an alkyl alkylimidate with dihydroxyacetone to give 2-alkyl-5-hydroxymethylimidazoles. Subsequent N-alkylarylation and oxidation yields 1-alkylaryl-2-alkyl-5-formylimidazoles. Although this process produces the key imidazole intermediates necessary for preparing the angiotensin II receptor antagonizing imidazoles described therein, the high pressure step limits the quantity of compound that can be produced using this method. Therefore, there is a need for an alternate method for the preparation of the imidazole intermediates on a commercial scale.

A further challenge in developing an alternate process is the fact that the regiospecific synthesis of N-substituted imidazoles is not a straight forward operation. Few syntheses exist which result in the exclusive formation of 1,2,5-substitution on the imidazole ring.

US-A-4482723 describes a process for preparing 4-acetyl-2-substituted imidazoles and novel intermediates used in the process.

It has now been found that the substituted 5-formylimidazole intermediates can be prepared by reacting a 2-halo-2-propenal-3-alkyl ether,-3-alkyl thioether, or -3-amine with a N-(1-iminoalkyl)aminoalkylaryl compound to produce said intermediates efficiently in high yield and high purity. The efficiency of the process and the quality and yields of the imidazole intermediates are particularly important when preparing compounds on a large scale for therapeutic use.

### DESCRIPTION OF THE INVENTION

The present invention provides a process for the preparation of a compound of formula (I): 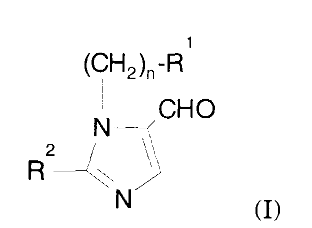 wherein:
R¹ is hydrogen, phenyl, biphenyl, or naphthyl, with each group being unsubstituted or substituted by one to three substituents selected from Cl, Br, F, I, C₁-C₆alkyl, nitro, A-CO₂R⁶, tetrazol-5-yl, SO₂NHR⁶, NHSO₂R⁶, SO₃H, CONR⁶R⁶, CN, SO₂C₁-C₆alkoxy, C₁-C₆alkoxy, hydroxy, SC₁-C₆alkyl, PO(OR⁶)₂, NR⁶R⁶, NR⁶CHO, NR⁶COC₁-C₆alkyl, NR⁶CON(R⁶)₂, NR⁶COW, W, and SO₂W;
R² is hydrogen, C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁶R⁶, CO₂R⁶, CN, CONR⁶R⁶, W, tetrazol-5-yl, NR⁶COC₁-C₆alkyl, NR⁶COW, SC₁-C₆alkyl, SO₂W, or SO₂C₁-C₆alkyl;
W is C_{q}F_{2q+1}, wherein q is 1-3;
A is -(CH₂)ₙ-, -CH=CH-, -O(CR⁴R⁵)ₘ-, or -S(CR⁴R⁵)ₘ-;
each R⁴, R⁵ independently is hydrogen, C₁-C₆alkyl (unsubstituted or substituted by phenyl, biphenyl, naphthyl or C₃-C₆cycloalkyl), phenyl, biphenyl, or naphthyl (each of which is unsubstituted or substituted by one to three substituents selected from Cl, Br, I, F, C₁-C₆alkyl, (alkenyl of up to 5 carbon atoms)CH₂, (alkynyl of up to 5 carbon atoms)CH₂, C₁-C₆alkoxy, C₁-C₆alkylthio, NO₂, CF₃, CO₂R⁶, or OH), C₃-C₆cycloalkyl, or phenyl(C₁-C₂alkyl) unsubstituted or substituted by phenyl;
each R⁶ independently is hydrogen, C₁-C₆alkyl, or (CH₂)ₙphenyl;
each n independently is 0-4; and
each m independently is 1-4;
or a pharmaceutically acceptable salt thereof, which comprises reacting a compound of formula (II): 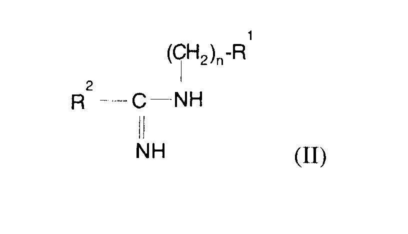 wherein:
R¹, R² and n are as defined above for formula (I), with a compound of formula (III): 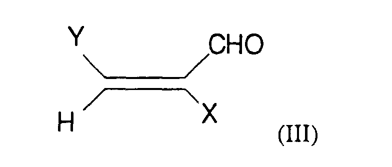
wherein:
X is Cl, Br, F, or I; and
Y is -OR³, -SR³, or -N(R³)₂, wherein R³ is C₁-C₆alkyl, under basic conditions and in solvent and, thereafter, optionally forming a pharmaceutically acceptable salt.

Preferably, the process can be used to prepare compounds of formula (I) in which:
R¹ is phenyl, biphenyl, or naphthyl, with each group being unsubstituted or substituted by one to three substituents selected from Cl, Br, F, CF₃, C₁-C₆alkyl, nitro, CO₂R⁶, OCR⁴R⁵CO₂R⁶, tetrazol-5-yl, C₁-C₆alkoxy, hydroxy, CN, or SO₂NHR⁶;
n is 1 or 2; and
R² is C₂-C₈alkyl.

It should be noted that, as used herein, the terms alkyl, alkenyl, alkoxy and alkynyl mean carbon chains which are branched or unbranched with the length of the chain determined by the descriptor preceding the term. Also, the term alkylaryl means -(CH₂)ₙR¹ wherein R¹ and n are as defined for formula (I) compounds.

In particular, the process can be used to prepare compounds of formula (I) in which R¹ is phenyl or naphthyl substituted by CO₂R⁶, preferably CO₂H, n is 1, and R² is C₂-C₈alkyl, preferably n-butyl. Most particularly, the process can be used to prepare 4-[(2-n-butyl-5-formyl-1H-imidazol-1-yl)methyl]benzoic acid and 4-[(2-n-butyl-5-formyl-1H-imidazol-1-yl)methyl] naphthoic acid.

Suitably, the reaction is carried out on compounds of formula (II) in which R¹, R², and n are as required in the desired formula (I) product. Preferably, the process is conducted with formula (II) compounds in which R¹ is phenyl or naphthyl substituted by CO₂R⁶, preferably CO₂H, n is 1, and R² is C₂-C₈alkyl, preferably n-butyl.

Suitably, the reaction is carried out on compounds of formula (III) in which X is Cl, Br, F, or I, preferably Br, and Y is -O-C₁-C₆alkyl, preferably iso-propyloxy.

Preferably, the reaction is carried out by reacting a 2-halo-2-propenal-3-alkyl ether, such as 2-bromo-3-(1-methylethoxy)-2-propenal, with a N-(1-iminoalkyl)aminoalkylaryl compound, such as N-(1-iminopentyl)-4-(aminomethyl)benzoic acid or N-(1-iminopentyl)-4-(aminomethyl)naphthoic acid, in the presence of base, such as an inorganic base, for example, sodium or potassium carbonate, or sodium or potassium hydroxide, preferably potassium carbonate, in solvent, such as water/organic solvent mixture, for example, water and tetrahydrofuran, water and acetonitrile, or water and chloroform containing 1, 4, 7, 10, 13, 16-hexaoxacyclooctadecane (18-Crown-6), preferably water and tetrahydrofuran. Suitably, the reaction is carried out at a temperature of between about 10°C and about 80°C, preferably between about 25°C and about 65°C.

Alternately, the reaction is carried out in the presence of an organic base and in an organic solvent. For example, a 2-halo-2-propenal-3-alkyl ether, such as 2-bromo-3-(1-methylethoxy)-2-propenal, is reacted with a N-(1-iminoalkyl)aminoalkylaryl compound, such as ethyl N-(1-iminopentyl)-4-(aminomethyl)benzoate or ethyl N-(1-iminopentyl-4-(aminomethyl)naphthoate, in the presence of an organic base, for example, triethylamine, diisopropylethylamine, or dimethylaminopyridine, preferably triethylamine, in an organic solvent, such as chlorinated hydrocarbons, for example, chloroform, dichloromethane, or 1,2-dichloroethane, preferably chloroform. Suitably, the reaction is carried out at a temperature of between about 10°C and about 80°C, preferably between about 25°C and about 65°C.

Alternately, the reaction is carried out using the N-(1-iminoalkyl)aminoalkylaryl compounds of formula (II) as the base. For example, a 2-halo-2-propenal-3-alkyl ether, such as 2-bromo-3-(1-methylethoxy)-2-propenal, is reacted with a N-(1-iminoalkyl)aminoalkylaryl compound, such as ethyl N-(1-iminopentyl)-4-(aminomethyl)benzoate or ethyl N-(1-iminopentyl)-4-(aminomethyl)naphthoate, in the presence of a catalytic amount of acetic acid, in an organic solvent, such as chlorinated hydrocarbons, for example, chloroform, dichloromethane, or 1,2-dichloroethane, preferably chloroform. Suitably, the reaction is carried out at a temperature of between about 10°C to about 80°C, preferably between about 25°C and about 65°C.

The starting N-(1-iminoalkyl)aminoalkylaryl compounds of formula (II) are prepared by reacting an alkyl alkylimidate, R²C(=NH)-O-C₁-C₆alkyl, for example, methyl valerimidate, with an aminoalkylaryl compound, such as 4-(aminomethyl)benzoic acid.

The starting 2-halo-2-propenal alkyl ether compounds of formula (In) are prepared by halogenation and deprotection of malonaldehyde bisdialkyl acetal, followed by O-alkylation of the 2-halo-malonaldehyde intermediate.

The invention is illustrated by the following example. The example is not intended to limit the scope of this invention as defined hereinabove and as claimed hereinbelow.

### Example 1

### Preparation of 4-[(2-n-Butyl-5-formyl-1H-imidazol-1-yl)methyl]benzoic Acid

### i. Preparation of methyl valerimidate hydrochloride

A 10 gallon, glass-lined fixed reactor was charged with 7.0 kg (84.6 mol) of valeronitrile and 2.96 kg (92.2 mol, 1.1 eq) of methanol. The solution was stirred with cooling to about 5°C under an atmosphere of nitrogen. A flow of hydrogen chloride gas from a gas cylinder was bubbled into the solution below the surface of the mixture at a rate such that the reaction temperature did not exceed 15°C. After about one hour, 3.67 kg (101 mol, 1.19 eq) of hydrogen chloride had been disbursed from the gas cylinder and addition was stopped. Stirring was continued for an additional 18 h at 0°C. Tert-butyl methyl ether (9.7 kg) was added to the suspension and stirring was continued for 3 h at 0°C. The slurry was then centrifuged under an atmosphere of nitrogen. After drying overnight under nitrogen and for several hours under reduced pressure at ambient temperature the product weighed 9.66 kg (76% yield uncorrected for purity) and had a mp of 91-92°C. The crude product was hygroscopic and was stored in sealed bottles under nitrogen at -5°C.

### ii. Preparation of N-(1-iminopentyl)-4-(aminomethyl)benzoic acid

A 22 L, three-necked round bottom flask equipped with an air-powered mechanical stirrer was placed under a nitrogen atmosphere. The vessel was charged with methyl valerimidate hydrochloride (2.5 kg, 16 mol) and dimethylformamide (9.2 L). A thermometer was attached and the suspension cooled to 0-15°C with a cooling bath. Triethylamine (2.3 L) was added to the reaction at such a rate so that the internal temperature did not exceed 25°C. The cooling was stopped and the reaction was allowed to stir 1 hour. The reaction mixture was vacuum filtered using a Büchner funnel and a carboy (20 L). The filter cake was washed with additional dimethylformamide (1.0 L) and force-air dried for 15 min. The combined filtrates were saved. Another clean 22 L, three-necked round bottom flask equipped as above was placed under nitrogen. The vessel was charged with the combined filtrates from above followed by triethylamine (1.6 L) and 4-(aminomethyl)benzoic acid (1.7 kg, 11.5 mol). The thermometer was attached and the suspension was heated to an internal temperature of 65°C with a heating mantle and a temperature controller. The heating was continued for 20 hours. The reaction was cooled to ambient temperature and filtered to yield 2.5 kg of product; 92% uncorrected yield.

### iii. Preparation of 2-bromo-malonaldehyde

A 12 L, three-necked round bottom flask was equipped with an air-powered mechanical stirrer with shaft, paddle, adapter, and thermometer was charged with 2.75 L of water and 110 mL of 12 N hydrochloric acid (1.32 mol). The addition funnel was charged with 2.5 kg of malonaldehyde bis(dimethyl acetal) (15.24 mol) which was then added to the stirred aqueous mixture in one portion. Stirring was continued for 30 min and a clear solution resulted. The reaction mixture was then cooled to 5°C using an ice-water bath. A 1 L addition funnel was charged with 790 mL of bromine (15.34 mol) and added to the reaction mixture at a rate such that the temperature did not exceed 25°C (approximately 30 min). The cooling bath was removed and the reaction mixture was allowed to stir at ambient temperature 1 hour. The reaction mixture is colorless to slightly yellow at this point. The solution was transferred to a 10 L round bottom flask and concentrated on the rotary evaporator at aspirator pressure (water bath 40°C) to approximately one-half the original volume. The reaction suspension was removed from the rotary evaporator and cooled at 10°C for 18 hour. Using a benchtop Büchner funnel and carboy (20 L), the resultant slurry was vacuum filtered. The solid was washed with 50% aqueous methanol (0.50 L) and force-air dried 2 hours. The mother liquor was returned to the 10 L round bottom flask and concentrated to approximately one-half its original volume. The flask was removed from the rotary evaporator and cooled (10°C) for 18 hours where additional solid emerged (331 g). The combined dried product was transferred to glass jars for storage to avoid contact with metal and was stored under refrigeration. This material was used as obtained; (2.0 kg, 86% uncorrected yield).

### iv. Preparation of 2-bromo-3-(1-methylethoxy)-2-propenal

With moderate agitation, a 20 gallon reactor system was charged with cyclohexane (29.12 L), 2-bromo-malonaldehyde (2.33 kg), p-toluenesulfonic acid monohydrate 43.94 g, and 2-propanol (4.65 L). The contents of the reactor were heated to allow for the removal of distillate under atmospheric pressure (jacket temp. 95°C and process temp. at 66.4°C). A total of 16 L of distillate was removed from the reaction via the cooling tower. This represents approximately 47% of the total volume of cyclohexane/2-propanol (33.77 L) being removed from the reactor. The reaction solution was cooled to near ambient temperature and transferred to a 10 gallon reactor system at 40°C. An additional 6 L of distillate was removed under vacuum (-64 torr, jacket temp. 62°C, and reaction temp. 25°C). The mobile dark orange oil was drained from the vessel and transferred to a rotary evaporator receiver flask and further concentrated under house vacuum at -30°C using a rotary evaporator. About 0.2 L more of solvent was removed. Total product obtained was 3.072 kg (16 mol, 103% yield). The product was used as obtained in the next step. This material is unstable and must be kept in a freezer (<-5°C, under nitrogen). The shelve-life is about 2 weeks.

### v. Preparation of 4-[(2-butyl-5-formyl-1H-imidazol-1-yl)methyl]benzoic acid

A 10 gallon, glass-lined fixed reactor was charged sequentially under nitrogen gas with tetrahydrofuran (17.96 L), N-(1-iminopentyl)-4-(aminomethyl)benzoic acid (2.2 kg, 9.4 mol), potassium carbonate (1.94 kg), and water (2.19 L). The suspension was then stirred. 2-bromo-3-(1-methylethoxy)-2-propenal (1.99 kg, 10.3 mol) was added in one portion using -0.3 L tetrahydrofuran as rinse. The stirred mixture was heated to reflux (63°C). Reflux was continued for 3 hours additional amounts of 2-bromo-3-(1-methylethoxy)-2-propenal (0.36 kg, 0.2 mol) was added to the vessel using 0.1 L tetrahydrofuran as rinse. After 4.0 hours reflux, additional 2-bromo-3-(1-methylethoxy)-2-propenal (0.18 kg, 0.1 mol) was added to the vessel using 0.1 L tetrahydrofuran as rinse. After 7.0 hours total reflux time, the reaction was cooled to 25°C and allowed to stand overnight with stirring. Water (3.6 L) was added to the vessel to dissolve any solids present and the solution was stirred 15 min. The solution was transferred to a 20 gallon, glass-lined fixed reactor. The original reactor was rinsed with 0.36 L of water which was also added to the 20 gallon vessel. This vessel was charged with ethyl acetate (21.5 L) and the suspension was stirred for 5 min and then allowed to settle. The dark aqueous alkaline product layer was transferred to a carboy (20 L) then added to a gallon vessel. The 20 gallon vessel was charged with water (2.9 L) and the suspension was stirred 5 min then allowed to settle. The bottom aqueous layer was collected and added to the 10 gallon vessel while the top ethyl acetate layer was collected for disposal. The basic (pH 10.05) aqueous solution was acidified with 6 N hydrochloric acid (2.51 L) to pH 5.2 then was transferred to a 20 gallon vessel. The 10 gallon vessel was rinsed with methylene chloride (26 L) and added to the 20 gallon vessel. The contents of the vessel were stirred for 10 min and the layers allowed to separate. The lower organic layer was transferred to a carboy (20 L). The 20 gallon vessel was charged with 4.3 L of methylene chloride and stirred for 5 min and then allowed to settle. After the phases separated, the bottom phase was collected in a carboy. The procedure was repeated once more with 4.3 L of methylene chloride. The combined methylene chloride extracts were added to a 10 gallon vessel and water was added (2.9 L). The suspension was stirred for 5 min then allowed to settle. The bottom organic layer was collected and placed in a portable 50 L glass tank. Under fast agitation, 0.67 kg of magnesium sulfate and 0.13 kg of activated charcoal were added and the suspension was filtered through a Büchner funnel containing Celite® under vacuum. The 10 gallon vessel was charged with the methylene chloride solution and the solvent was removed under vacuum until about 5 L remained. The reactor was then charged with 2-butanone (5.17 L) and solvent was removed under vacuum until the volume was 4-5 L. Ethyl acetate was added (13 L) and the suspension was stirred 16 hours. At this time, the solid was removed by filtration using a Büchner funnel under vacuum. The collected solid was rinsed with a mix of 2-butanone:ethyl acetate (10:90) and dried overnight under vacuum. The yield obtained was 1457 g (5.08 mol, 94.0% purity).

It is to be understood that the invention is not limited to the embodiment illustrated hereinabove and the right to the illustrated embodiment and all modifications coming within the scope of the following claims is reserved.

## Claims

1. A process for the preparation of a compound of formula (I): 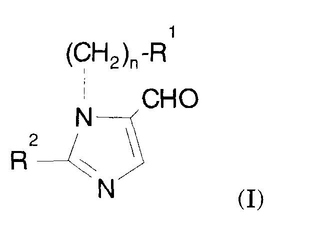 wherein:
R¹ is hydrogen, phenyl, biphenyl, or naphthyl, with each group being unsubstituted or substituted by one to three substituents selected from Cl, Br, F, I, C₁-C₆alkyl, nitro, A-CO₂R⁶, tetrazol-5-yl, SO₂NHR⁶, NHSO₂R⁶, SO₃H, CONR⁶R⁶, CN, SO₂C₁-C₆alkoxy, C₁-C₆alkoxy hydroxy, SC₁-C₆alkyl, PO(OR⁶)₂, NR⁶R⁶, NR⁶CHO, NR⁶COC₁-C₆alkyl, NR⁶CON(R⁶)₂, NR⁶COW, W, and SO₂W;
R² is hydrogen, C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁶R⁶, CO₂R⁶, CN, CONR⁶R⁶, W, tetrazol-5-yl, NR⁶COC₁-C₆alkyl, NR⁶COW, SC₁-C₆alkyl, SO₂W, or SO₂C₁-C₆alkyl;
W is C_{q}F_{2q+1}, wherein q is 1-3;
A is -(CH₂)ₙ-, -CH=CH-, -O(CR⁴R⁵)ₘ-, or -S(CR⁴R⁵)ₘ-;
each R⁴, R⁵ independently is hydrogen, C₁-C₆alkyl (unsubstituted or substituted by phenyl, biphenyl, naphthyl or C₃-C₆cycloalkyl), phenyl, biphenyl, or naphthyl (each of which is unsubstituted or substituted by one to three substituents selected from Cl, Br, I, F, C₁-C₆alkyl, (alkenyl of up to 5 carbon atoms)CH₂, (alkynyl of up to 5 carbon atoms)CH₂, C₁-C₆alkoxy, C₁-C₆alkylthio, NO₂, CF₃, CO₂R⁶, or OH), C₃-C₆cycloalkyl, or phenyl(C₁-C₂alkyl) unsubstituted or substituted by phenyl;
each R⁶ independently is hydrogen, C₁-C₆alkyl, or (CH₂)ₙphenyl;
each n independently is 0-4; and
each m independently is 1-4;
or a pharmaceutically acceptable salt thereof, which comprises reacting a compound of formula (II) : 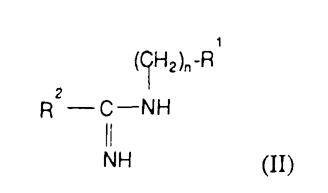 wherein:
R¹, R² and n are as defined above for formula (I), with a compound of formula (III): 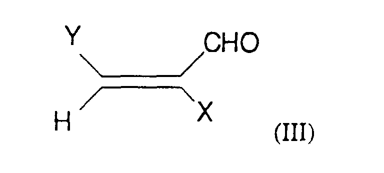
wherein:
X is Cl, Br, F, or I; and
Y is -OR³, -SR³, or -N(R³)₂, wherein R³ is C₁-C₆alkyl, under basic conditions and in solvent and, thereafter, optionally forming a pharmaceutically acceptable salt.

2. The process of claim 1 for preparing a compound wherein:
R¹ is phenyl, biphenyl, or naphthyl, with each group being unsubstituted or substituted by one to three substituents selected from Cl, Br, F, CF₃, C₁-C₆alkyl, nitro, CO₂R⁶, -OCR⁴R⁵CO₂R⁶, tetrazol-5-yl, C₁-C₆alkoxy, hydroxy, CN, or SO₂NHR⁶;
n is 1 or 2; and
R² is C₂-C₈alkyl.

3. The process of claim 2 for preparing a compound wherein:
R¹ is phenyl or naphthyl substituted by CO₂R⁶;
n is 1; and
R² is C₂-C₈alkyl.

4. The process of claim 3 for preparing a compound which is 4-[(2-n-butyl-5-formyl-1H-imidazol-1-yl)methyl]benzoic acid.

5. The process of claim 3 for preparing a compound which is 4-[(2-n-butyl-5-formyl-1H-imidazol-1-yl)methyl]naphthoic acid.

6. The process of claims 4 and 5 in which the base is potassium carbonate and the solvent is water and tetrahydrofuran.

7. The process of claim 3 for preparing a compound which is ethyl 4-[(2-n-butyl-5-formyl-1H-imidazol-1-yl)methyl]benzoate.

8. The process of claim 3 for preparing a compound which is ethyl 4-[(2-n-butyl-5-formyl-1H-imidazol-1-yl)methyl]naphthoate.

9. The process of claims 7 and 8 in which the base is triethylamine and the solvent in chloroform.

## Patentansprüche

1. Verfahren zur Herstellung einer Verbindung der Formel (I): 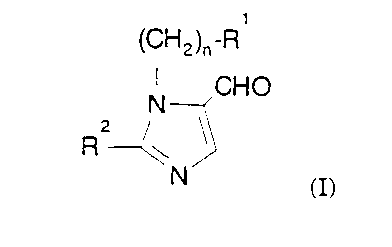 in der
R¹ Wasserstoff, Phenyl, Biphenyl oder Naphthyl ist, wobei jede Gruppe unsubstituiert oder durch einen bis drei aus Cl, Br, F, I, C₁-C₆-Alkyl, Nitro, A-CO₂R⁶, Tetrazol-5-yl, SO₂NHR⁶, NHSO₂R⁶, SO₃H, CONR⁶R⁶, CN, SO₂C₁-C₆-Alkoxy, C₁-C₆-Alkoxyhydroxy, SC₁-C₆-Alkyl, PO(OR⁶)₂, NR⁶R⁶, NR⁶CHO, NR⁶COC₁-C₆-Alkyl, NR⁶CON(R⁶)₂, NR⁶COW, W und SO₂W ausgewählten Substituenten substituiert ist;
R² Wasserstoff, C₂-C₁₀-Alkyl, C₃-C₁₀-Alkenyl, C₃-C₁₀-Alkinyl, C₃-C₆-Cycloalkyl oder (CH₂)₀₋₈-Phenyl ist, das unsubstituiert oder mit einem bis drei aus C₁-C₆-Alkyl, Nitro, Cl, Br, F, I, Hydroxy, C₁-C₆-Alkoxy, NR⁶R⁶, CO₂R⁶, CN, CONR⁶R⁶, W, Tetrazol-5-yl, NR⁶COC₁-C₆-Alkyl, NR⁶COW, SC₁-C₆-Alkyl, SO₂W oder SO₂C₁-C₆-Alkyl ausgewählten Substituenten substituiert ist;
W für C_{q}F_{2q+1} steht, wobei q für 1 bis 3 steht:
A für -(CH₂)ₙ-, -CH=CH-, -O(CR⁴R⁵)ₘ- oder -S(CR⁴R⁵)ₘ- steht;
jeder Rest R⁴, R⁵ unabhängig voneinander Wasserstoff, C₁-C₆-Alkyl (unsubstituiert oder mit Phenyl, Biphenyl, Naphthyl oder C₃-C₆-Cycloalkyl substituiert), Phenyl, Biphenyl oder Naphthyl (die jeweils unsubstituiert oder mit einem bis drei aus Cl, Br, I, F, C₁-C₆-Alkyl, (Alkenyl mit bis zu 5 Kohlenstoffatomen)-CH₂, (Alkinyl mit bis zu 5 Kohlenstoffatomen)-CH₂, C₁-C₆-Alkoxy, C₁-C₆-Alkylthio, NO₂, CF₃, CO₂R⁶ oder OH ausgewählten Substituenten substituiert sind), C₃-C₆-Cycloalkyl oder Phenyl(C₁-C₂-Alkyl), das unsubstituiert oder mit Phenyl substituiert ist, bedeutet;
jeder Rest R⁶ unabhängig voneinander Wasserstoff, C₁-C₆-Alkyl oder (CH₂)ₙ-Phenyl ist;
jedes n unabhängig voneinander 0 bis 4 ist und
jedes m unabhängig voneinander 1 bis 4 ist,
oder eines pharmazeutisch verträglichen Salzes davon, umfassend die Umsetzung einer Verbindung der Formel (II) 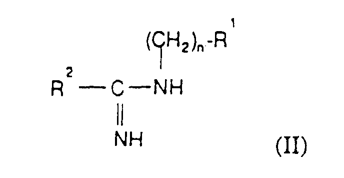 in der
R¹, R² und n die gleiche Bedeutung haben wie vorstehend für die Formel (I) definiert
mit einer Verbindung der Formel (III) 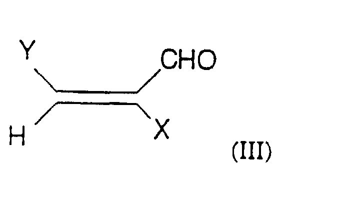 in der
X für Cl, Br, F oder I steht und
Y für -OR³, -SR³ oder -N(R³)₂ steht, wobei R³ ein C₁-C₆-Alkyl ist,
unter basischen Bedingungen und in einem Lösungsmittel und anschließend ggfs. die Bildung eines pharmazeutisch verträgliches Salzes.

2. Verfahren nach Anspruch 1 zur Herstellung einer Verbindung, in der
R¹ Phenyl, Biphenyl oder Naphthyl ist, wobei jede Gruppe unsubstituiert oder mit einem bis drei aus Cl, Br, F, CF₃, C₁-C₆-Alkyl, Nitro. CO₂R⁶, -OCR⁴R⁵CO₂R⁶, Tetrazol-5-yl, C₁-C₆-Alkoxy, Hydroxy, CN oder SO₂NHR⁶ ausgewählten Substituenten substituiert ist,
n für 1 oder 2 steht und
R² für C₂-C₈-Alkyl steht.

3. Verfahren nach Anspruch 2 zur Herstellung einer Verbindung, in der
R¹ Phenyl oder mit CO₂R⁶ substituiertes Naphthyl ist;
n für 1 steht und
R² für C₂-C₈-Alkyl steht.

4. Verfahren nach Anspruch 3 zur Herstellung einer Verbindung, bei der es sich um 4-[(2-n-Butyl-5-formyl-1H-imidazol-1-yl)methyl]benzoesäure handelt.

5. Verfahren nach Anspruch 3 zur Herstellung einer Verbindung, bei der es sich um 4-[(2-n-Butyl-5-formyl-1H-imidazol-1-yl)methyl]naphthoesäure handelt.

6. Verfahren nach Anspruch 4 und 5, bei dem die Base Kaliumcarbonat und das Lösungsmittel Wasser und Tetrahydrofuran ist.

7. Verfahren nach Anspruch 3 zur Herstellung einer Verbindung, bei der es sich um 4-[(2-n-Butyl-5-formyl-1H-imidazol-1-yl)methyl]benzoesäureethylester handelt.

8. Verfahren nach Anspruch 3 zur Herstellung einer Verbindung, bei der es sich um 4-[(2-n-Butyl-5-formyl-1H-imidazol-1-yl)methyl]naphthoesäureethylester handelt.

9. Verfahren nach Anspruch 7 und 8, bei dem die Base Triethylamin und das Lösungsmittel Chloroform ist.

## Revendications

1. Procédé pour la préparation d'un composé de formule (I) : 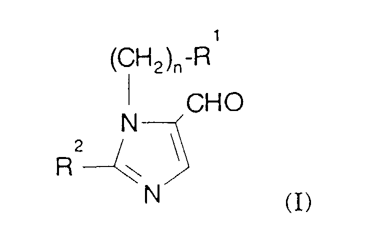 dans laquelle :
R¹ représente l'hydrogène, un groupe phényle, biphényle ou napthyle, chaque groupe étant substitué ou non substitué avec un à trois substituants choisis entre des substituants Cl, Br, F, I, alkyle en C₁ à C₆, nitro, A-CO₂R⁶, tétrazole-5-yle, SO₂NHR⁶, NHSO₂R⁶, SO₃H, CONR⁶R⁶, CN, SO₂(alkoxy en C₁ à C₆), alkoxy en C₁ à C₆, hydroxy, S(alkyle en C₁ à C₆), PO(OR⁶)₂, NR⁶R⁶, NR⁶CHO, NR⁶CO(alkyle en C₁ à C₆), NR⁶CON(R⁶)₂, NR⁶COW, W et SO₂W ;
R² représente l'hydrogène, un groupe alkyle en C₂ à C₁₀, alcényle en C₃ à C₁₀, alcynyle en C₃ à C₁₀, cycloalkyle en C₃ à C₆ ou (CH₂)₀₋₈phényle, non substitué ou substitué avec un à trois substituants choisis entre des substitants alkyle en C₁ à C₆, nitro, Cl, Br, F, I, hydroxy, alkoxy en C₁ à C₆, NR⁶R⁶, CO₂R⁶, CN, CONR⁶R⁶, W, tétrazole-5-yle, NR⁶CO(alkyle en C₁ à C₆), NR⁶COW, S(alkyle en C₁ à C₆), SO₂W ou SO₂(alkyle en C₁ à C₆) ;
W représente un groupe C_{q} F_{2q+1}, dans lequel q a une valeur de 1 à 3 ;
A représente un groupe -(CH₂)ₙ-, -CH=CH-, -O(CR⁴R⁵)ₘ- ou -S(CR⁴R⁵)ₘ- ;
chacun des groupes R⁴ et R⁵ représente indépendamment l'hydrogène, un groupe alkyle en C₁ à C₆ (non substitué ou substitué avec un substituant phényle, biphényle, naphtyle ou cycloalkyle en C₃ à C₆), phényle, biphényle ou naphtyle (chacun non substitué ou substitué avec un à trois substituants choisis entre des substituants Cl, Br, I, F, alkyle en C₁ à C₆, (alcényle ayant jusqu'à 5 atomes de carbone) CH₂, (alcynyle allant jusqu'à 5 atomes de carbone)CH₂, alkoxy en C₁ à C₆, alkylthio en C₁ à C₆, NO₂, CF₃, CO₂R⁶ et OH), cycloalkyle en C₃ à C₆ ou phényle(alkyle en C₁ ou C₂) non substitué ou substitué avec un substituant phényle ;
chaque groupe R⁶ représente indépendamment l'hydrogène, un groupe alkyle en C₁ à C₆, ou (CH₂)ₙphenyle ;
chaque indice n a un indépendamment une valeur de 0 à 4 ; et
chaque indice m a indépendamment une valeur de 1 à 4 ;
ou d'un de ses sels pharmaceutiquement acceptables, qui comprend la réaction d'un composé de formule (II) : 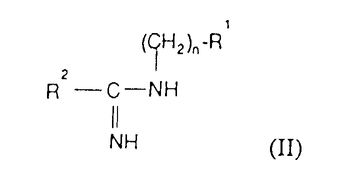 dans laquelle :
R¹, R² et n répondent aux définitions précitées pour la formule (I),
avec un composé de formule (III) : 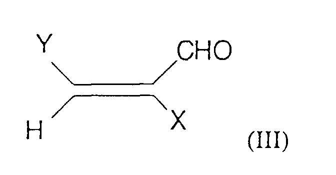 dans laquelle :
X représente Cl, Br, F ou I ; et
Y représente un groupe -OR³, -SR³ ou -N(R³)₂, dans lequel R³ représente un groupe alkyle en C₁ à C₆, en milieu basique et dans un solvant, puis, facultativement, la formation d'un sel pharmaceutiquement acceptable.

2. Procédé suivant la revendication 1, pour la préparation d'un composé dans lequel :
R¹ représente un groupe phényle, biphényle ou naphtyle, chaque groupe étant non substitué ou substitué avec un à trois substituants choisis entre des substituants Cl, Br, F, CF₃, alkyle en C₁ à C₆, nitro, CO₂R⁶, -OCR⁴R⁵CO₂R⁶, tétrazole-5-yle, alkoxy en C₁ à C₆, hydroxy, CN et SO₂NHR⁶ ;
n est égal à 1 ou 2 ; et
R² représente un groupe alkyle en C₂ à C₈.

3. Procédé suivant la revendication 2, pour la préparation d'un composé dans lequel :
R¹ représente un groupe phényle ou naphtyle substitué avec un substituant CO₂R⁶ ;
n est égal à 1 ; et
R² représente un groupe alkyle en C₂ à C₈.

4. Procédé suivant la revendication 3 pour la préparation d'un composé qui consiste en l'acide 4-[(2-n-butyl-5-formyl-1H-imidazole-1-yl)méthyl]benzoïque.

5. Procédé suivant la revendication 3 pour la préparation d'un composé qui consiste en l'acide 4-[(2-n-butyl-5-formyl-1H-imidazole-1-yl)méthyl]naphtoïque.

6. Procédé suivant les revendications 4 et 5, dans lequel la base consiste en carbonate de potassium et le solvant consiste en eau et tétrahydrofuranne.

7. Procédé suivant la revendication 3 pour la préparation d'un composé qui consiste en 4-[(2-n-butyl-5-formyl-1H-imidazole-1-yl)méthyl]benzoate d'éthyle.

8. Procédé suivant la revendication 3 pour la préparation d'un composé qui consiste en 4-[(2-n-butyl-5-formyl-1H-imidazole-1-yl)méthyl]naphtoate d'éthyle.

9. Procédé suivant les revendications 7 et 8, dans lequel la base consiste en triéthylamine et le solvant consiste en chloroforme.
